(19) Europäisches Patentamt
European Patent Office
Office européen des brevets

(11) Veröffentlichungsnummer: **0 172 515**
**B1**

# (12) EUROPÄISCHE PATENTSCHRIFT

(45) Veröffentlichungstag der Patentschrift: **13.05.87**

(51) Int. Cl.⁴: **C 07 C 121/45**, C 07 D 239/42

(21) Anmeldenummer: **85110089.1**

(22) Anmeldetag: **12.08.85**

(54) **Alpha-(o-chlorphenyl)-aminomethylen-beta-formylaminopropionitril, Verfahren zu seiner Herstellung sowie Verwendung zur Herstellung von 2-Methyl-4-amino-5-formylaminomethylpyrimidin.**

(30) Priorität: **25.08.84 DE 3431270**

(43) Veröffentlichungstag der Anmeldung: **26.02.86 Patentblatt 86/9**

(45) Bekanntmachung des Hinweises auf die Patenterteilung: **13.05.87 Patentblatt 87/20**

(84) Benannte Vertragsstaaten: **CH DE FR GB IT LI NL**

(56) Entgegenhaltungen: **EP - A - 0 001 760**

(73) Patentinhaber: **BASF Aktiengesellschaft, Carl-Bosch-Strasse 38, D-6700 Ludwigshafen (DE)**

(72) Erfinder: **Ernst, Hansgeorg, Dr., Bruesseler Ring 38, D-6700 Ludwigshafen (DE)**
Erfinder: **Littmann, Wolfgang, Dr., Neckarpromenade 36, D-6800 Mannheim 1 (DE)**
Erfinder: **Paust, Joachim, Dr., Ringstrasse 3, D-6708 Neuhofen (DE)**

**EP 0 172 515 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein spezielles N-substituiertes α-Aminomethylen-β-formylaminopropionitril, das zur Herstellung von 2-Methyl-4-amino-5-formylaminomethyl-pyrimidin II dient, und seine Herstellung aus Metallsalzen des α-Formyl-β-formylaminopropionitrils III. Die Verbindung II ist ein wichtiges Zwischenprodukt zur Herstellung von Vitamin $B_1$.

Aus der europäischen Patentschrift 0001760 ist ein vorteilhaftes Verfahren zur Herstellung von 2-Methyl-4-amino-5-formylaminomethylpyrimidin II

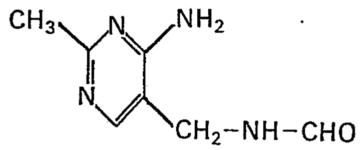

bekannt, indem man Salze des α-Formyl-β-formylaminopropionitrils III

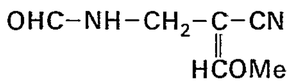

wobei Me ein Kation, vorzugsweise ein Alkali oder Erdalkalikation bedeutet, mit einem Salz des Ammoniaks oder eines Amins der Formel

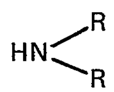

in der die Reste R Wasserstoffatome oder gleiche oder verschiedene Alkyl, Aryl oder Aralkylreste bedeuten oder zusammen mit dem Stickstoffatom einen heterocyclischen Ring bilden können, zu den neuen Verbindungen α-Aminomethylen-β-formylaminopropionitril der Formel I

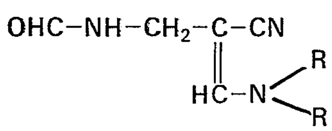

umsetzt, in der R die obengenannte Bedeutung hat, und diese Verbindungen mit Acetamidin zum Pyrimidin II cyclisiert.

Als aromatische Aminkomponente ist dort N-Methylanilin und Anilin genannt, wobei insbesondere die Anilinoverbindung als Ausgangsmaterial für die weitere Umsetzung zum Pyrimidin der Formel II beschrieben ist.

Es wurde nun überraschenderweise gefunden, dass besonders hohe Ausbeuten bei gleichzeitiger Verkürzung der Reaktionszeit an dem Pyrimidin der Formel II

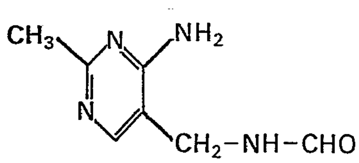

erhalten werden, wenn man α-(o-Chlorphenyl)-aminomethylen-β-formylaminopropionitril der Formel I'

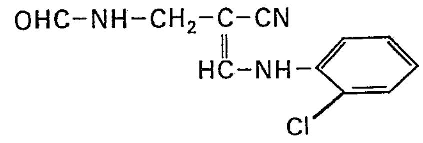

mit Acetamidin cyclisiert.

Die Verbindung der Formel I' wird in an sich aus EP-B 0001760 bekannter Weise erhalten, wenn man Salze des α-Formyl-β-formylaminopropionitrils III

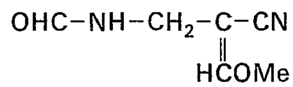

wobei Me ein Kation, vorzugsweise ein Alkali oder Erdalkalikation bedeutet, mit einem Salz des ortho-Chloranilins in wässerigen Medium umsetzt.

Bezüglich der Herstellung der Ausgangsstoffe der Formel III wird auf EP-B 0001760 verwiesen, auf deren Angaben hiermit Bezug genommen wird.

Als Salze des o-Chloranilins kommen z. B. das Sulfat oder die Salze der Halogenwasserstoffsäuren, insbesondere das Hydrochlorid in Betracht.

Die Umsetzung eines Salzes des o-Choranilins mit α-Formyl-β-formylaminopropionitril erfolgt zweckmässig in wässerigen Medium, wobei das o-Chloranilinsalz als Suspension vorgelegt und das Natriumsalz des α-Formyl-β-formylaminopropionitrils bei normaler oder leicht erhöhter Temperatur z. B. bis zu 40°C zugegeben wird. Man erhält so unmittelbar die gewünschte Verbindung der Formel I' in kristalliner Form.

Die weitere Umsetzung der neuen Verbindung der Formel I' mit Acetamidin geschieht in der in der deutschen Offenlegungsschrift 23 23 845 für das Ausgangsmaterial α-Alkoxymethylen-β-formylaminopropionitril beschriebenen Weise zu dem Pyrimidin II. Die Reaktion kann in Gegenwart eines inerten Lösungsmittels durchgeführt werden. Als Lösungsmittel eignen sich die in der deutschen Offenlegungsschrift 23 23 845 aufgeführten Lösungsmittel sowie weitere inerte Lösungsmittel wie beispielsweise Acetonitril und Dimethylformamid sowie auch das bei der Umsetzung freiwerdende o-Chloranilin selbst.

Dabei erhält man unter Verwendung des neuen α-(o-Chlorphenyl)-aminomethylen-β-formylaminopropionitril um ca. 8% höhere Ausbeute als unter Verwendung der entsprechenden p-Chloranilinoverbindung und sogar um ca. 15 bis 20% höhere Ausbeuten als unter Verwendung der entsprechenden Anilinoverbindung, Verkürzung der Reaktionszeit von 18 bis 20 Stunden auf 30 bis 60 Minuten, und benötigt einen geringeren Acetamidinüberschuss.

*Beispiel 1:*

a) Eine Mischung von 382,5 g (3 mol) o-Chloranilin und 600 ml Wasser wird unter Kühlung mit Wasser mit 200 ml konzentrierter Salzsäure versetzt. Dann tropft man unter Rühren innerhalb etwa 30 Min. eine Lösung von 444 g des Natriumsalzes von α-Formyl-β-formylaminopropionitril (90,65%ig ≙ 2,72 mol) in 1500 ml Wasser bei 25 bis 30°C zu. Gleichzeitig wird der pH durch Zugabe von konzentrierter Salzsäure bei 1,3 gehalten.

Man rührt über Nacht bei Raumtemperatur nach, saugt ab, wäscht zweimal mit je ca. 1 l Wasser und trocknet den Filterkuchen über Nacht bei +50°C im Vakuumtrockenschrank.

Auswaage: 625,2 g (97,6% d. Th.) α-(o-Chlorphenyl)-aminomethylen-β-formylaminopropionitril.

Das Produkt ist NMR-spektroskopisch rein.

b) 23,6 g (0,10 mol) des wie vorstehend erhaltenen Enamins werden in 20 ml Acetonitril vorgelegt. Bei 50°C werden innerhalb von 20 Min. 13,4 g Acetamidin (86,4%ig. ≙ 0,20 mol) zugetropft. Es erfolgt ein langsamer Temperaturanstieg auf 61°C; die Temperatur sinkt innerhalb von ca. 30 Min. wieder auf +50°C. Man rührt noch etwa 15 Min. bei +50°C nach, so dass die gesamte Reaktionszeit 60 Min. beträgt. Man lässt das Reaktionsgemisch auf Raumtemperatur kommen, gibt 20 ml Diethylketon zu, rührt kurz auf, filtriert ab, wäscht den Filterkuchen zweimal mit je 50 ml Diethylketon und trocknet das Produkt im $N_2$-Strom.

Auswaage: 14,27 g (86,0% d. Th.) Formylaminomethyl-aminopyrimidin.

Fp.: 219-220°C

Reinheit: 95,9%

c) 23,6 g (0,01 mol) Enamin gemäss Absatz a) werden in 20 ml Acetonitril vorgelegt. Bei 50°C werden innerhalb von 20 Min. 13,4 g Acetamidin (enthält 13,6 Gew.-% MeOH; ≙ 0,20 mol) zugetropft.

Man rührt 5 Min. bei 50°C nach, wobei die Innentemperatur auf + 62°C steigt. Dann wird Acetonitril im Wasserstrahlvakuum abgezogen, und man rürht die Mischung unter vermindertem Druck nochmals 45 Min., wobei die Temperatur des Reaktionsgemisches bei +50°C gehalten wird. Die Gesamt-Reaktionszeit nach Beendigung der Acetamidinzugabe beträgt 1 Stunde. Dann gibt man 80 ml Isopropanol zu, erhitzt 10 Min. unter Rückflusskühlung, kühlt auf +5°C ab, rührt 15 Min. bei +5°C nach, saugt ab, wäscht zweimal mit je 50 ml Isopropanol nach und trocknet den Filterkuchen im $N_2$-Strom.

Auswaage: 13,85 g (83,4% d. Th.) Formylaminomethyl-aminopyrimidin.

Fp.: 219-221°C

Reinheit: 98,8%

*Beispiel 2:*

a) Zu einer Mischung aus 31,9 g (0,25 mol) o-Chloranilin und 50 ml Wasser werden 9 ml konzentrierte Schwefelsäure gegeben. Anschliessend tropft man unter Rühren eine Lösung von 41,1 g des in Beispiel 1 beschriebenen Na-Salzes (90,65%ig; ≙ 0,25 mol) innerhalb einer Stunde zu. Man rührt 23 h bei Raumtemperatur nach, saugt ab, wäscht viermal mit je 100 ml Wasser und trocknet im Vakuumtrockenschrank bei +40°C.

Auswaage: 54,5 g (92,6% d. Th.) α-(o-Chlorphenyl)-aminomethyl-β-formylaminopropionitril.

Fp.: 124-126°C

b) 23,6 g (0,1 mol) des gemäss (a) erhaltenen Enamins werden mit 13,05 g Acetamidin (88,9%ig; ≙ 0,20 mol) gemischt und die Mischung wird auf 50°C erhitzt. Wenn das Gemisch ca. 50°C erreicht, setzt die exotherme Reaktion ein, und die Temperatur steigt innerhalb weniger Minuten auf etwa 90°C. Nach einer Gesamtreaktionszeit von ca. 25 Min. gibt man nun 100 ml Diethylketon zu, rührt 15 Min. bei +50°C nach, lässt auf Raumtemperatur abkühlen, saugt ab und wäscht den Filterkuchen zweimal mit je 50 ml Diethylketon. Das Produkt wird im $N_2$-Strom getrocknet.

Auswaage: 14,53 g (87,5% d. Th.) Formylaminomethyl-aminopyrimidin.

*Beispiel 3:*

Zu einer Mischung aus 25,47 g (0,20 mol) o-Chloranilin und 40 ml Wasser werden 14 ml konzentrierte Salzsäure gegeben. In 15 Min. wird unter Rühren zu dieser Suspension eine Lösung von 32,85 g des in Beispiel 1 beschriebenen Na-Salzes (90,3%ig = 0,20 mol) in 100 ml Wasser zugetropft. Durch gleichzeitige Zugabe von konzentrierter Salzsäure wird dabei ein pH-Wert von 1,5 aufrechterhalten. Man rührt über Nacht bei Raumtemperatur nach, saugt ab, wäscht dreimal mit je 100 ml Wasser und trocknet im Vakuumtrockenschrank bei +40°C.

Auswaage: 43,8 g = 93,0% d. Th. α-(o-Chlorphenyl)-aminomethyl-β-formylaminopropionitril.

*Beispiel 4:*

a) Eine Mischung aus 63,79 g (0,50 mol) o-Chloranilin und 100 ml Wasser wird mit konzentrierter Salzsäure auf pH 1,2 eingestellt. Zu dieser Suspension lässt man unter Rühren die Lösung von 74,0 g des in Beispiel 1 beschriebenen Na-Salzes (90,3%ig = 0,452 mol) in 250 ml Wasser innerhalb von 20 Min. zulaufen (Temperaturanstieg bis 37°C). Unter gleichzeitiger Zugabe von konzentrierter Salzsäure wird dabei ein pH-Wert von 1,2 aufrechterhalten.

Man rührt über Nacht bei Raumtemperatur nach, saugt ab, wäscht dreimal mit je 150 ml Wasser und trocknet den Filterkuchen bei 40°C im Vakuumtrockenschrank.

Auswaage: 99,5 g (93,5% d. Th.) α-(o-Chlorphenyl)-aminomethylen-β-formylaminopropionitril.

b) 27,61 g Acetamidin (84%ig, das entspricht 0,40 mol) werden auf 50°C erwärmt. Man gibt innerhalb von 3 Min. portionsweise 47,1 g (0,20 mol) des Enamins gemäss Absatz a) zu. Nach erfolgter Durchmischung setzt eine exotherme Reaktion ein (Temperaturanstieg auf 110°C); man läss ausreagieren und rührt noch 22 h bei 50°C nach. Dann gibt man 150 ml Diethylketon zu und rührt die Mischung 30 Min. bei 50°C. Man lässt auf Raumtemperatur abkühlen, saugt ab und wäscht zweimal mit je 80 ml Diethylketon. Der Filterkuchen wird im $N_2$-Strom getrocknet.

Auswaage: 31,71 g (95,5% d. Th.) Formylaminomethyl-aminopyrimidin.

Reinheit: 89%

c) In einem weiteren Ansatz werden zu 6,42 g Acetamidin (84%ig ≙ 0,093 mol) bei +50°C 11,77 g (0,050 mol) des o-Chlorenamins gemäss Absatz a) in 2 Min. portionsweise zugegeben. Nach 5 Min. steigt die Temperatur des Reaktionsgemisches in etwa 3 Min. auf 88°C an und sinkt anschliessend wieder auf +50°C. Insgesamt belässt man das Gemisch 1 h bei einer Badtemperatur von 50°C; dann gibt man 40 ml Diethylketon zu und rührt 30 Min. bei +50°C nach. Man saugt ab, wäscht zweimal mit je 50 ml Diethylketon und trocknet den Filterkuchen im $N_2$-Strom.

Auswaage: 7,85 g (94,6% d. Th.) Formylaminomethyl-aminopyridimin.

Reinheit: 90,2%

d) 3,53 g Acetamidin (84%ig; ≙ 0,051 mol) werden bei 50°C portionsweise mit 11,77 g (0,050 mol) des Enamins gemäss Absatz a) und anschliessend mit 1 ml Dioxan versetzt. Man rührt 4 h bei 50°C nach, gibt 40 ml Diethylketon zu und rührt nochmals 30 Min. bei 50°C nach.

Dann wird abgesaugt, zweimal mit je 50 ml Diethylketon gewaschen und getrocknet.

Auswaage: 6,90 g (83,1% d. Th.) Formylaminomethyl-aminopyrimidin.

*Vergleichsbiespiel 5:*

a) 23,44 g (0,252 mol) Anilin werden mit 25 ml 36%iger Salzsäure vermischt. Die Suspension wird durch Zugabe von 20 ml Wasser gelöst. Innerhalb von 30 Min. wird eine Lösung von 41,66 g des in Beispiel 1 beschriebenen Natriumsalzes (88,8%ig; ≙ 0,250 mol) zugetropft. Dabei wird mit konzentrierter Salzsäure ein pH-Wert von 2,0-2,5 eingehalten. Dann wird 23 h bei Raumtemperatur nachgerührt und abgesaugt. Der Filterkuchen wird viermal mit je 100 ml Wasser gewaschen und im Vakuumtrockenschrank bei +45°C getrocknet.

Auswaage: 46,4 g (92,3% d. Th.) α-Phenylaminomethylen-β-formylaminopropionitril.

b) Ausbeute an 2-Methyl-4-amino-5-formylaminomethylpyrimidin aus α-Phenylaminomethylen-β-formylaminopropionitril und deren Zeitabhängigkeit:

13,05 g Acetamidin (88,9%ig; ≙ 0,20 mol) werden bei 50°C mit 20,0 g (0,1 mol) des Enamins gemäss vorstehendem Absatz versetzt. Man rührt bei 50°C nach, gibt dann 80 ml Isopropanol zu, erhitzt 10 Min. unter Rückfluss, kühlt auf 5°C ab, rührt 15 Min. bei 5°C nach und saugt ab. Der Filterkuchen wird zweimal mit je 50 ml Isopropanol gewaschen und im $N_2$-Strom getrocknet.

| | | Formylaminomethyl-aminopyrimidin | | |
|---|---|---|---|---|
| Nachrührzeit bei 50°C h | Auswaage g | Ausbeute % | Fp. (°C) | Reinheit |
| 5 | 9,44 | 56,9% | 221 -221,5 | 96,3% |
| 7 | 10,04 | 60,5% | 220 -221 | |
| 16 | 11,10 | 66,9% | 220,5-221 | |
| 18 | 11,32 | 68,2% | 221 -221,5 | 96,6% |
| 24 | 11,22 | 67,6% | 221 -221,5 | 96,0% |
| 48 | 11,32 | 68,2% | 221,5-222 | 97,0% |

Reduziert man den Acetamidinüberschuss auf 50 mol-%, sinkt die Ausbeute an Pyrimidinderivat um 15-20%. Setzt man 1 Äquivalent Acetamidin ein, wird die Ausbeute halbiert.

**Patentansprüche**

1. α-(o-Chlorphenyl)-aminomethylen-β-formylaminopropionitril.

2. Verfahren zur Herstellung der Verbindung gemäss Anspruch 1, dadurch gekennzeichnet, dass man Metallsalze von α-Formyl-β-formylaminopropionitril mit Salzen des o-Chloranilins in wässerigen Medium umsetzt und das Enamin isoliert.

3. Verwendung von α-(o-Chlorphenyl)-aminomethylen-β-formylaminopropionitril zur Herstellung der Verbindung der Formel

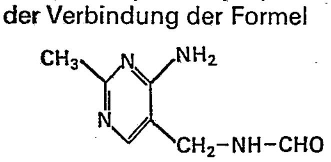

durch Umsetzung mit Acetamidin.

**Claims**

1. α-(o-Chlorophenyl)-aminomethylene-β-formylaminopropionitrile.

2. A process for the preparation of the compound as claimed in Claim 1, wherein a metal salt of α-formyl-β-formylaminopropionitrile is reacted with a salt of o-chloroaniline in aqueous medium, and the enamine is isolated.

3. The use of α-(o-chlorophenyl)-aminomethylene-β-formylaminopropionitrile for the preparation of the compound of the formula

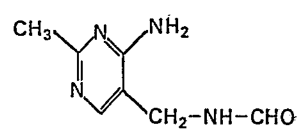

by reaction with acetamidine.

**Revendications**

1. α-(o-Chlorophényl)-aminométhylène-β-formylaminopropionitrile.

2. Procédé de préparation du composé selon la revendication 1, caractérisé en ce qu'on fait réagir des sels métalliques d'α-formyl-β-formylamino-propionitrile avec des sels de l'o-chloraniline en milieu aqueux et on isole l'énamine.

3. Utilisation d'α-(o-chlorophényl)-aminométhylène-β-formylaminopropionitrile pour la préparation du composé de formule

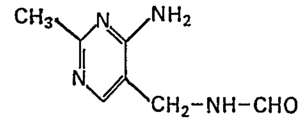

par réaction avec l'acétamidine.